# EUROPEAN PATENT APPLICATION

(11) **EP 1 745 747 A1**
(43) Date of publication of application: **24.01.2007**
(21) Application number: 04732738.2
(22) Date of filing: 13.05.2004
(51) Int. Cl.: A61B 17/00, A61M 11/00

(54) **APPARATUS FOR HEMOSTASIS AND ADHESION PREVENTION FOR USE IN ENDOSCOPIC SURGERY**

(71) Applicant: Nakanishi Inc., Kanuma-shi, Tochigi 322-8666 (JP)
(72) Inventor: KAWATA, Sousaku, c/o NAKANISHI INC., Kanuma-shi, Tochigi3228666 (JP)
(74) Representative: Wehnert, Werner
(86) International application number: PCT/JP2004/006452
(87) International publication number: WO 2005/115252

(57) **Abstract**

The present invention relates to a system for hemostasis and prevention of adhesion for use in endoscopic surgery which allows quick and precise transfer of biopolymer particles in a constant amount onto a surgical site without agglomeration. The system utilizes a powder agitating container including a biopolymer case, a noninflammable gas supply line for supplying a noninflammable gas into the case, and a mixture transfer line for transferring a mixture of the noninflammable gas and the biopolymer. The container is fixed to the control box, the mixture is sprayed through a spray nozzle, and a gelling solution is sprayed from near the mixture.

## Description

The present invention relates to a system for spraying fine biopolymer powder, which is used for hemostasis or prevention of visceral adhesion in endoscopic surgery.

Biocompatible biopolymers, such as oxidized cellulose, carboxymethyl cellulose, hyarulonic acid, collagen, and the like, have conventionally been used in application to surgical sites during surgery or wound sites for the purpose of hemostasis, prevention of adhesion, prevention of keloid, wound treatment, or close-up or sealing of cuts. Such biopolymers are in the form of fiber sheets, films, granules, or gels. However, such forms as sheets or the like prevent application of the biopolymers in hemostasis or prevention of adhesion in the body cavity or of surgical sites after endoscopic surgery due to lack of enough space.

In view of this, JP-2003-62057-A discloses technology that allows precise adhesion and arrangement of a biopolymer irrespective of the size, shape, and location of the application site. A biopolymer is made into fluidized fine particles under the injection force of a gas, which particles are sprayed with a gas injecting agent (noninflammable gas) onto a surgical site in the body cavity or of post-endoscopic surgery.

However, the invention disclosed in this publication is directed to sprayable fine biopolymer particles for hemostasis or prevention of adhesion, 80% of which are in the particle size range of up to 100 µm in the particle size distribution, which has the average particle size of not larger than 50 µm, which may be fluidized with a gas, and which may be used for hemostasis, prevention of adhesion, prevention of keloid, wound treatment, close-up or sealing precisely at an application site, irrespective of the size, shape, and location of the application site. No spraying system for practical application of the fine particles has been found.
Patent Publication 1: JP-2003-62057-A

It is an object of the present invention to provide a system for gelling and spraying a biopolymer as a device for hemostasis and prevention of adhesion for use in endoscopic surgery, which system allows quick spraying, in a constant amount, of the fine biopolymer particles disclosed in the above patent publication, with a solution, in as loose a state as possible without agglomeration, onto a surgical site.

The present invention has achieved the above object, and the gist thereof lies in a system for hemostasis and prevention of adhesion for use in endoscopic surgery comprising a powder agitating container including a powder agitating container for accommodating fine biopolymer particles therein, a noninflammable gas supply line arranged in a rear part of said powder agitating container for supplying a noninflammable gas to mix with said biopolymer in the container, and a mixture transfer line arranged in a front part of said powder agitating container for transferring a mixture of said biopolymer and said noninflammable gas,
wherein said powder agitating container is fixed to a control box,
wherein a powder transfer tube is arranged for spraying said mixture from said mixture transfer line through a spray nozzle provided at an end of a grip for an operator onto a surgical site, and
wherein a solution tube is arranged for transferring a solution contained in a solution bottle, along said powder transfer tube,
whereby said mixture is sprayed from near the spray nozzle spraying said solution.

The fine biopolymer particles in the powder agitating container are uniformly mixed and agitated in the container by a noninflammable gas transferred through a noninflammable gas supply line arranged in the rear part of the container fixed to the control box, and a constant amount of the particles are sprayed onto a surgical site via a mixture transfer line and a powder transfer line, through a spray nozzle, together with a solution transferred through another line.

Thus a surgeon, as an operator, after endoscopic surgery, may stanch bleeding or prevent adhesion simply by spraying the fine biopolymer particles with a gas through the powder agitating container onto a surgical site, and the nonuniform spraying of the biopolymer or clogging of tubes may be prevented.

The biopolymer used with the present invention may be one or more of hemostatic, anti-adhesion, biocompatible polymers, such as carboxymethyl cellulose, carboxyethyl cellulose, oxidized cellulose, chitin, chitosan, hyarulonic acid, starch, glycogen, arginate, pectin, dextran, chondroitin sulfate, gelatine,and collagen. The noninflammable gas to be mixed with the polymer for transferring the same may be carbon dioxide gas, nitrogen gas, or the like, and the solution used for gelling the transferred polymer may be saline.

According to the present invention as set forth in claim 2, the spray nozzle has a structure for spraying through the center thereof a mixture of the biocompatible polymer and the noninflammable gas, and, substantially simultaneously therewith, spraying from around the mixture a gelling solution transferred through a line separately from the mixture. By starting spraying of the mixture slightly later than the solution, the mixture may be made to adhere to the surgical site without scattering around, whereas by stopping spraying of the mixture slightly later than the solution, so-called sagging at the surgical site may be prevented.

According to the present invention as set forth in claim 3, the powder agitating container for accommodating a biopolymer, the powder transfer tube, the spray nozzle with the grip, and the solution tube extending from the solution bottle containing saline or the like up to the spray nozzle, are made disposable so as to prevent in-hospital infection of pathogens.

According to the present invention as set forth in claim 4, the powder agitating container, the powder transfer tube, and the spray nozzle with the grip are packed in a sterile bag, for convenience of storage and use in surgery, and for ensuring hygiene.

According to the present invention as set forth in claim 5, the powder transfer tube and the gelling solution tube are flexible tubes for facilitating control of the spray nozzle with the grip by the operator. In addition, by fixing the powder agitating container to the control box, unlike a conventional powder agitating container held and used as a dental handpiece, the inlet of the mixture transfer line in the powder agitating container may always be positioned in the upper part of the container, so that the biopolymer in the container is ensured to be completely transferred by the noninflammable gas.

According to the present invention as set forth in claim 6, the noninflammable gas supply line is provided with an electrostatic grounding for preventing static build-up in the line up to the powder agitating container. The interior surface of the powder agitating container is coated with anon-electrostatic resin,suchasteflon (registeredtrade mark), and the mixture transfer line is made of an antistatic material treated with teflon (registered trade mark), for eliminating obstacles caused by electrostatic charge in transfer of the mixture up to the spray nozzle.

In the present invention, a mixture of the biopolymer and the noninflammable gas transferred through the mixture transfer line is sprayed through the spray nozzle, whereas the gelling solution transferred through the gelling solution tube distinct from the mixture transfer line is separately sprayed through the spray nozzle. Further, the biopolymer is sprayed with the noninflammable gas in a constant amount. Thus clogging is prevented from the container body up to the spray nozzle.

The spray nozzle is structured so that the discharge port for the mixture is arranged in the center, and the discharge port for the solution is arranged around the mixture discharge port. With this structure, scattering of the mixture at the treatment site may be prevented as much as possible.

The powder agitating container, the powder transfer tube as a powder transfer line, the spray nozzle with the grip, and the gelling solution tube extending from the solution bottle to the spray nozzle are made disposable. Thus in-hospital infection may be prevented.

The powder agitating container, the powder transfer tube, and the spray nozzle with the grip are packed in a sterile bag, for use one bag at a time. Thus storage conditions until use in surgery are improved.

The powder transfer tube and the gelling solution tube are made flexible, which contributes to improved handling by an operator and convenience in endoscopic surgery.

The noninflammable gas supply line is provided with an electrostatic grounding, the powder agitating container is lined with a non-electrostatic resin, and the mixture transfer line is made of an antistatic material, which allows uniform diffusion and uniform spraying of the biopolymer.

In endoscopic surgery, the powder agitating container is used, and the powder transfer tube extending from the biopolymer/noninflammable gas mixture transfer line is provided separately from the solution tube extending from the solution bottle for separately spraying the mixture and the solution through the spray nozzle.

The present invention will now be explained with reference to examples taken in conj unction with the attached drawings.

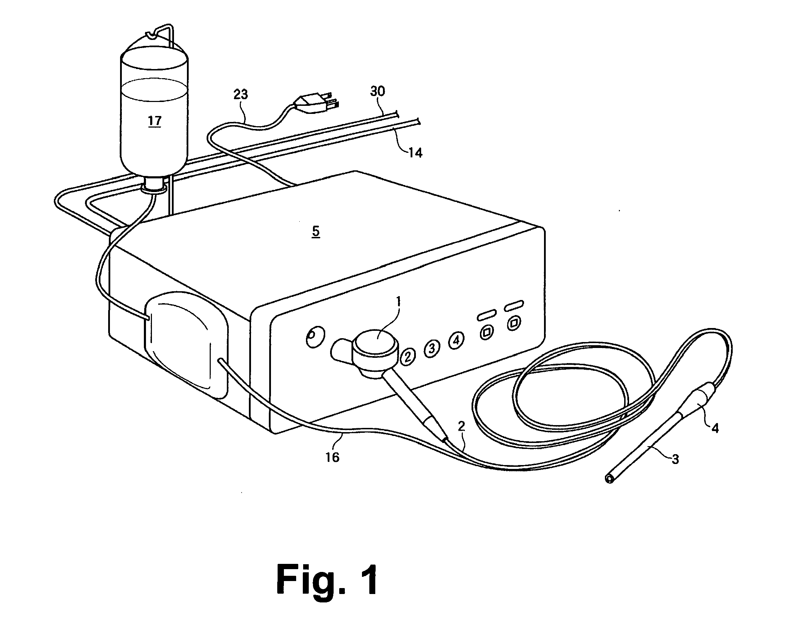

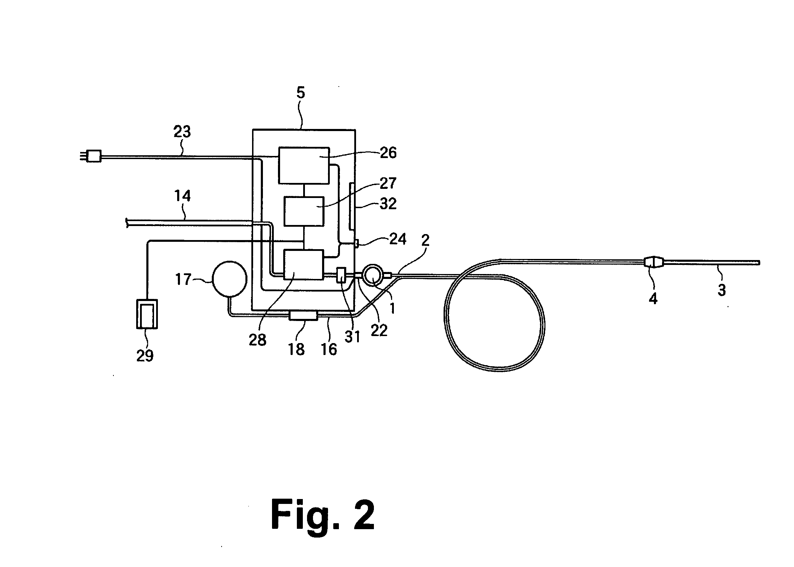

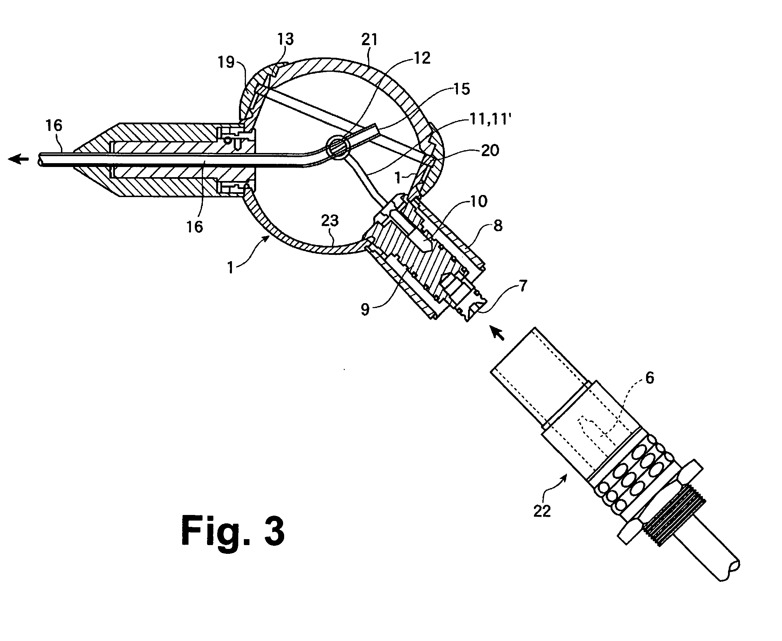

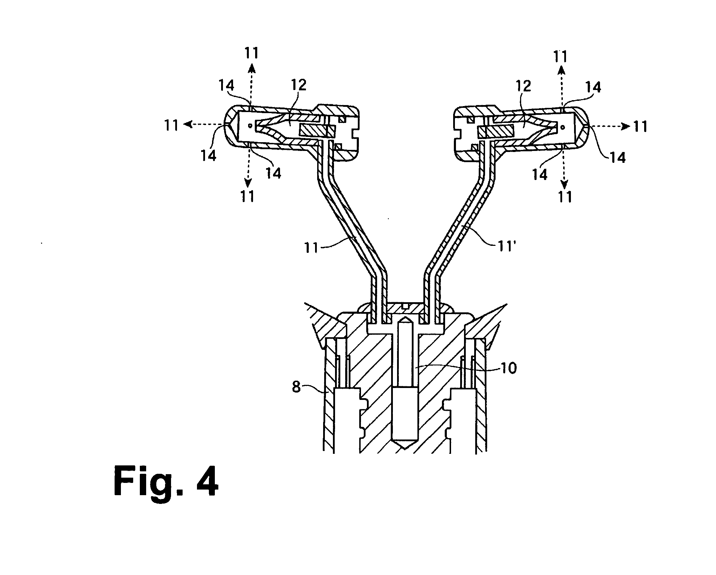

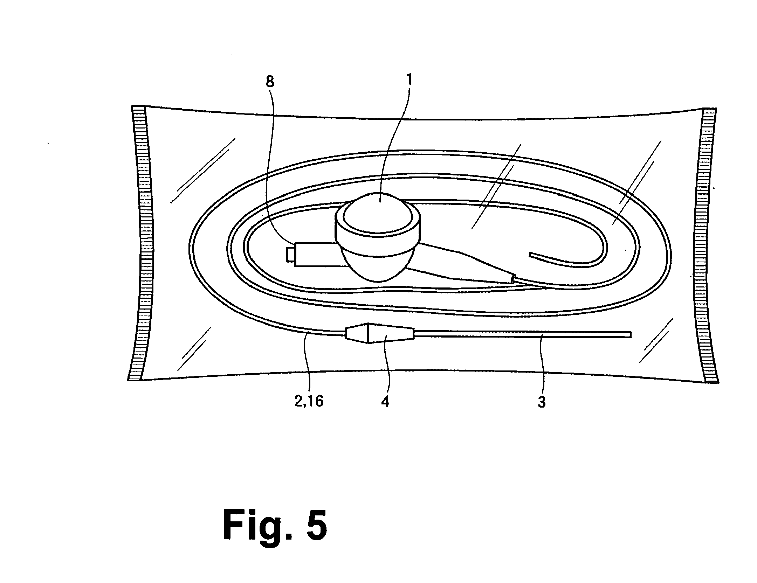

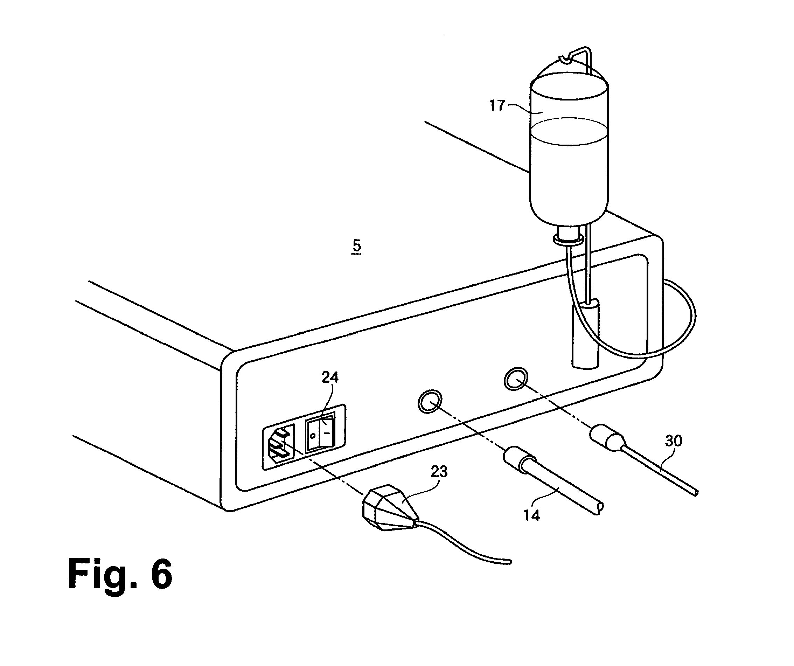

Fig. 1 is an outline view of the entire system according to the present invention. Fig. 2 is a schematic explanatory view of the internal mechanism of the system of Fig. 1. Fig. 3 is a longitudinal sectional view of the powder agitating container of Figs. 1 and 2. Fig. 4 is a partial plan view of the noninflammable gas transfer line of Fig. 3. Fig. 5 is an explanatory view showing the powder agitating container to which the spray nozzle with the grip is attached via the powder transfer tube, all packed in a sterile bag. Fig. 6 is a perspective view of the rear side of the system of Fig. 1.

In the figures, 1 denotes a powder agitating container, and at the forward end thereof, spray nozzle 3 with grip 4 is attached via powder transfer tube 2. The powder agitating container 1 is connected to control box 5 by inserting lock key 6 projected in connector 22 which is fixed in the control box 5, into lock keyway 7 provided in connection plug 9 which is projected in sleeve 8 at the proximal end of the container 1. By this connection, an internal duct in the lock key 6 is communicated with gas supply line 10 leading from the lock keyway 7. A noninflammable gas 14, such as carbon dioxide gas, from a gas cylinder (not shown) is lead through gas input plug 14, lock key 6, lock keyway 7, gas supply line 10, and bifurcate gas lines 11, 11' branching from the gas supply line 10, and passed through a gas discharge nozzle for powder agitation equipped with a duck bill backflow prevention valve (referred to simply as check valve hereinbelow) 12, which nozzle is provided at the end of each bifurcate gas line. The gas is distributed to five outlet ports 14 on each nozzle, arranged on the top, bottom, right, and left sides of the nozzle at 90° intervals and also in the direction exiting the check valve 12 (straight direction), i.e., distributed to in total of ten outlet ports 14, and sprayed therethrough into the powder agitating container 1.

The noninflammable gas 11 sprayed into the powder agitating container 1 through the outlet port 14 in the straight direction of each check valve 12, directly hits the left or right wall surface of the container 1 and scatters in every direction, whereas the gas 11 through the other outlet ports 14 on the top, bottom, right, and left sides of the same check valve 12 is also sprayed and scattered uniformly in vertical and horizontal directions along the right or left wall of the container 1. By this flow of the noninflammable gas 11, fine biopolymer particles (with the average particle size of not larger than 100 µm) filling about a half of the powder agitating container 1 are uniformly dispersed in the container 1, and continuously flown into fluidized polymer port 15 of tubular, mixture transfer line 16 positioned in the upper portion of the container 1. The mixture of the biopolymer and the noninflammable gas is sprayed, via the powder transfer tube 2 in the form of a flexible tube, through the spray nozzle 3 with the grip 4.

On the other hand, saline contained in solution bottle 17, which is suspended at the back of the control box 5, is transferred forward by means of solution pump 18, which forwards the saline by rubbing a tubular flow channel, and arranged in parallel with the mixture transfer line 16 in the form of the flexible tube as mentioned above. The saline is sprayed onto the application site in the body through the spray nozzle 3 from around the mixture being sprayed.

Incidentally, annular gaskets are arranged at two locations 13 and 20 in the upper part of the powder agitating container 1, and sealing cap 21 and container body 23 containing the biopolymer are sealed with lock nut 19 screwed thereon.

For operation of the system by an operator, first, power switch 24 is turned on to connect 100V power plug 23 to voltage regulator 26, control board 27, and automatic gas regulator 28 in the control box 5. By stepping on foot switch 29, the automatic gas regulator 28, via foot plug 30, is regulated in three-stage voltage adjustment, i.e., high, medium, and low, to regulate the flow rate of the noninflammable gas 11 from gas inlet plug 14 into the powder agitation container 1.

Further, biofilter 31 is disposed between the automatic gas regulator 28 and the connector 22 to filter the noninflammable gas 11 before entry into the powder agitating container 1 for sterilizing the mixture to be sprayed through the spray nozzle 3 as completely as possible. This biofilter 31 is exchageable after every use.

In this way, the automatic gas regulator 28 is controlled by stepping on the foot switch 29 to introduce the noninflammable gas 11 into the powder agitating container 1. The gas 11 is mixed with the fine biopolymer particles in the container body 23, and sprayed onto the treatment site, or stopped, through the spray nozzle 3 with the solution, to thereby stanch bleeding or prevent postoperative adhesion.

The flow rate of the noninflammable gas 11 into the container body 23 is regulated with the control box 5 to control the amount of the mixture to be sprayed onto the treatment site, to thereby selectively optimize the effect, such as hemostasis, depending on the condition. A preferred ratio between the mixture and the solution to be sprayed is about 7:3. It is preferred to properly gel the mixture at the treatment site and to prevent scattering of the mixture, by starting spraying the mixture slightly later than the solution, and stopping spraying the mixture slightly earlier than the solution.

The present invention may be applied to hemostasis and prevention of adhesion in endoscopic surgery.
Fig. 1 is an outline view of the entire system according to the present invention.
Fig. 2 is a schematic view of the internal mechanism of the system of Fig. 1.
Fig. 3 is a longitudinal sectional view of the powder agitating container of Figs. 1 and 2.
Fig. 4 is a partial plan view of the noninflammable gas transfer line in the sleeve of Fig. 3.
Fig. 5 is an explanatory view showing the powder agitating container to which the spray nozzle with the grip is attached via the powder transfer tube, all packed in a sterile bag.
Fig. 6 is an explanatory view of the rear side of the system of Fig. 1.

- 1: powder agitating container
- 2: mixture transfer line (transfer tube)
- 3: spray nozzle
- 4: grip
- 5: control box
- 11, 11': bifurcate gas line
- 12: backflow prevention valve
- 14: outlet port
- 15: fluidized polymer port
- 16: mixture transfer line
- 17: bottle of gelling solution
- 31: sterile bag

## Claims

1. A system for hemostasis and prevention of adhesion for use in endoscopic surgery comprising a powder agitating container including a powder agitating container for accommodating fine biopolymer particles therein, a noninflammable gas supply line arranged in a rear part of said powder agitating container for supplying a noninflammable gas to mix with said biopolymer in said container, and a mixture transfer line arranged in a front part of said container for transferring a mixture of said biopolymer and said noninflammable gas,
wherein said powder agitating container is fixed to a control box,
wherein a powder transfer tube is arranged for spraying said mixture from the mixture transfer line through a spray nozzle provided at an end of a grip for an operator onto a surgical site, and
wherein a solution tube is arranged along said powder transfer tube for transferring a solution contained in a solution bottle,
whereby said mixture is sprayed from near the spray nozzle spraying said solution.

2. The system for hemostasis and prevention of adhesion for use in endoscopic surgery according to claim 1, wherein said spray nozzle has a structure for spraying said solution from around the mixture through a solution discharge port, and wherein a controller is provided for starting spraying of the solution prior to start of spraying of the mixture, and stopping spraying of the mixture prior to termination of spraying of the solution.

3. The system for hemostasis and prevention of adhesion for use in endoscopic surgery according to claim 1, wherein said powder agitating container for accommodating the biopolymer, said powder transfer tube, said spray nozzle with the grip, and said solution tube from the solution bottle up to the spray nozzle, are disposable.

4. The system for hemostasis and prevention of adhesion for use in endoscopic surgery according to claim 3, wherein said powder agitating container, said powder transfer tube, and said spray nozzle with the grip are packed in a sterile bag.

5. The system for hemostasis and prevention of adhesion for use in endoscopic surgery according to claim 1, wherein said powder transfer tube and said solution tube are flexible.

6. The system for hemostasis and prevention of adhesion for use in endoscopic surgery according to claim 1, wherein said noninflammable gas supply line extending up to the powder agitating container is provided with an electrostatic grounding, the powder agitating container is lined with a non-electrostatic resin, and the mixture transfer line is made of an antistatic material.
